# EUROPÄISCHE PATENTSCHRIFT

(11) **EP 0 889 125 B1**
(45) Veröffentlichungstag und Bekanntmachung des Hinweises auf die Patenterteilung: **02.02.2005**
(21) Anmeldenummer: 98108668.9
(22) Anmeldetag: 13.05.1998
(51) Int. Cl.: C12N 15/06, C07K 16/28, C12N 5/20, G01N 33/577, G01N 33/574, A61K 39/395

(54) **Monoklonaler Antikörper 4G8B4B12 gegen humanes FLT3/FLK2**
Monoclonal antibody 4G8b4b12 to human FLT3/FLK2
Anticorps monoclonal 4G8B4B12 contre le FLT3/FLK2 humain

(30) Priorität: 30.06.1997 DE 19727814
(43) Veröffentlichungstag der Anmeldung: 07.01.1999
(73) Patentinhaber: Eberhard-Karls-Universität Tübingen Universitätsklinikum, 72076 Tübingen (DE)
(72) Erfinder: Bühring, Hans-Jörg, Dr., 72076 Tübingen (DE); Rappold, Irene, 72074 Tübingen (DE)
(74) Vertreter: Otten, Hajo, Dr.-Ing.

(56) Entgegenhaltungen:
- WO-A-95/07348
- DE-C- 19 608 769
- I. RAPPOLD ET AL.: "Epitope recognition, functional aspects, and reactivity of anti-FLT3-FLK2 antibodies." TISSUE ANTIGENS, Bd. 48, Nr. 4-2, Oktober 1996, Seite 399 XP002080668 Copenhagen, Denmark
- O. ROSNET ET AL.: "Human Flt3/Flk2 receptor tyrosine kinase is expressed at the surface of normal and malignant hematopoietic cells." LEUKEMIA, Bd. 10, Nr. 2, Februar 1996, Seiten 238-248, XP002080669 Baltimore, MD, USA
- N. GOLDSTEIN: "71E1, anti-flk-2 tyrosine kinase receptor." HYBRIDOMA, Bd. 14, Nr. 5, Oktober 1995, Seite 513 XP002080670 New York, NY, USA
- C. ROSE ET AL.: "Isolation and characterization of a monoclonal antibody binding to the extracellular domain of the flk-2 tyrosine kinase receptor." HYBRIDOMA, Bd. 14, Nr. 5, Oktober 1995, Seiten 453-459, XP002080671 New York, NY, USA
- I. RAPPOLD ET AL.: "Functional and phenotypic characterization of cord blood and bone marrow subsets expressing FLT3 (CD135) receptor tyrosine kinase." BLOOD, Bd. 90, Nr. 1, 1. Juli 1997, Seiten 111-125, XP002080672 New York, NY, USA

## Beschreibung

Die Erfindung betrifft einen Antikörper gegen den humanen Tyrosin-Kinase-Rezeptor FLT3/FLK2.

Der Tyrosin-Kinase-Rezeptor FLT3/FLK2 spielt eine wichtige Rolle bei der frühen Hämatopoese. Er kommt natürlicherweise in Blutstammzellen und in frühen lymphoiden und myeloischen Blutvorläuferzellen vor und interagiert mit einem Wachstumsfaktor FLT-3-Ligand (FL), der insbesondere die Rekrutierung und die Proliferation dieser Zellen stimuliert (Matthews et al., Cell 65:1143, 1991; Small et al., Proc. Natl. Acad. Sci. USA 91:459, 1994; Lyman et al., Blood 83:2795, 1994; Muench et al., Blood 85:963, 1995; Hannum et al., Nature 368:643, 1994; Hirayama et al., Blood 85:1762, 1995).

Der indirekte Nachweis vermittels eines Antikörpers, der spezifisch an den Rezeptor bindet, ist ein sicheres und schnelles Verfahren zum qualitativen und quantitativen Nachweis von membrangebundenen Rezeptoren. Dieser spezifische Antikörper kann entweder direkt oder indirekt markiert sein, wobei diese Markierung dazu benutzt wird, den Antikörper bzw. den damit verbundenen Rezeptor zu identifizieren und zahlenmäßig zu bestimmen. Als Marker kommen hier insbesondere Fluoreszenzfarbstoffe oder radioaktive Stoffe in Betracht.

Ein solcher Antikörper kann auch mit speziellen, therapeutisch wirksamen Reagenzien gekoppelt werden und damit die Möglichkeit einer zellulär zielgerichteten Behandlung bieten, die insbesondere bei Tumorerkrankungen zu fordern ist.

Es wurden bereits Antikörper gegen FLT3/FLK2-Rezeptorprotein beschrieben, diese sind jedoch spezifisch gegen das Maus-FLT3/FLK2-Rezeptorprotein gerichtet. Für eine Anwendung an menschlichen Zellen sind diese Antikörper deshalb nicht geeignet, denn es fehlt ihnen die diesbezügliche Spezifität.

Ein monoklonaler Antikörper, der spezifisch an das native, unmodifizierte humane FLT3/FLK2-Rezeptorprotein bindet, wird von Hybridomzellen produziert und freigesetzt, die unter der Nummer DSM ACC2248 bei der Deutschen Sammlung von Mikroorganismen und Zellkulturen GmbH (DSMZ) nach den Vorschriften des Budapester Vertrags hinterlegt worden sind. Er ist mit der Bezeichnung BV10A4H2 benannt. Dieser Antikörper ist Gegenstand der DE 196 08 769 "Antikörper BV10A4H2".

Rappold et al., "EPITOPE RECOGNITION, FUNCTIONAL ASPECTS, AND REACTIVITY OF ANTI-FLT3/FLK2 ANTIBODIES", Tissue Antigens (1996), Bd. 48, S. 399, nennt vier monoklonale Antikörper, die gegen die extrazelluläre Domäne der FLT3/FLK2-Rezeptor-Tyrosin-Kinase gerichtet sind. Sämtliche dieser Antikörper zeichnen sich durch unterschiedliche Affinitäten und antagonistische bzw. agonistische Eigenschaften aus.

In der WO 95/07348 A1 sind monoklonale Antikörper genannt, die gleichermaßen gegen die FLT3/FLK2-Rezeptor-Tyrosin-Kinase aus der Maus und dem Menschen gerichtet sind, mittels verschiedener Antigene erzeugt wurden, unterschiedliche Affinitäten und Spezifitäten haben und verschiedenen IgG-Klassen angehören.

Rosnet et al., "HUMAN FLT3/FLK2 RECEPTOR TYROSINE KINASE IS EX-PRESSED AT THE SURFACE OF NORMAL AND MALIGNENT HEMATOPOIETIC CELLS", Leukemia (1996), Bd. 10, 238, beschreiben die Gewinnung von bestimmten monoklonalen Antikörpern, gegen die menschliche FLT3/FLK2-Rezeptor-Tyrosin-Kinase.

Der vorliegenden Erfindung liegt die Aufgabe zugrunde, einen weiteren monoklonalen Antikörper bereitzustellen, der spezifisch an das native, unmodifizierte humane FLT3/FLK2-Rezeptorprotein bindet und der in praktisch unbegrenzter Menge zur Verfügung steht.

Diese Aufgabe wird durch die Bereitstellung eines monoklonalen Antikörpers gelöst, der spezifisch an das native, unmodifizierte humane FLT3/FLK2-Rezeptorprotein bindet und der von Hybridomzellen produziert und freigesetzt wird, die unter der Nummer DSM ACC 2249 bei der Deutschen Sammlung von Mikroorganismen und Zellkulturen GmbH (DSMZ) nach den Vorschriften des Budapester Vertrags hinterlegt worden sind. Er ist mit der Bezeichnung 4G8B4B12 benannt.

Mit dem erfindungsgemäßen Antikörper wurde erstmals jeweils ein monoklonaler Antikörper bereitgestellt, der standardisiert reproduzierbar ist und somit potentiell unbegrenzt hergestellt werden kann, und der spezifisch an jeweils ein spezielles extrazelluläres Epitop auf dem humanen Rezeptorprotein FLT3/FLK2 bindet.

Der erfindungsgemäße Antikörper ermöglicht eine gezielte Erkennung und Beeinflussung von Zellen, die eine extrazelluläre Domäne des humanen FLT3/FLK2-Rezeptorproteins aufweisen. Er stellt damit ein einzigartiges und vielseitig einsetzbares Mittel für den Arzt und Forscher dar, um einerseits solche Zellen nachzuweisen, und zwar sowohl in der Zellkultur als auch im Patientenorganismus, und um andererseits diese Zellen ggf. zu manipulieren, entweder durch den Antikörper selbst oder durch daran gekoppelte, spezifische Reagenzien.

Die Erfindung betrifft ferner Hybridomzellen, die einen monoklonalen Antikörper gegen das FLT3/FLK2-Rezeptorprotein erzeugen und die unter der Nummer DSM ACC2249 bei der Deutschen Sammlung von Mikroorganismen und Zellkulturen GmbH (DSMZ) nach dem Budapester Vertrag hinterlegt sind und den Antikörper mit der Bezeichnung 4G8B4B12 produzieren.

Ein Verfahren zur Herstellung von Hybridomzellen, die.einen Antikörper gegen das native, unmodifizierte FLT3/FLK2-Rezeptorprotein synthetisieren und freisetzen, umfaßt die im Stand der Technik grundsätzlich geläufigen Schritte, wie sie bspw. von Bühring et al. in Hybridoma 1991, Band 10, Nr. 1, S. 77-78 beschrieben wurden:
1. Immunisierung oder Sensibilisierung eines Tieres, vorzugsweise einer Maus vom Balb/c-Stamm, mit dem Antigen bzw. Immunogen;
2. Gewinnung der antikörperproduzierenden Zellen,vorzugsweise der Milzlymphozyten dieses Tieres;
3. Fusionierung dieser antikörperproduzierenden Zellen mit einer stabilen, immortalisierten Zellinie, vorzugsweise einer Myelomzellinie, zu Hybridomzellen; und
4. Vereinzelung und Vermehrung (Klonierung) solcher Hybridomzellen, die einen Antikörper sezernieren, der an das Antigen bindet.

Das Verfahren zeichnet sich dadurch aus, daß das Tier mit Zellen der Mauszellinie BA/F3, die zuvor mit der kompletten cDNA-Sequenz für den humanen FLT3/FLK2-Rezeptor transfiziert wurde (BA/F3-huFLT3), immunisiert wird.

Das hat den Vorteil, daß die Zellen dieser Zellinie BA/F3-huFLT3 eine starke Expression von FLT3/FLK2-Rezeptorprotein zeigen, wie sich während der zu diesen transfizierten BA/F3-huFLT3-Zellen führenden Versuche zeigte.

Bei der Suche nach Hybridomzellen, die FLT3/FLK2-Rezeptorprotein-spezifische Antikörper produzieren, ist es vorteilhaft, wenn nur solche vereinzelten und klonierten Hybridomzellen ausgewählt werden, die Antikörper produzieren, die eine Spezifität für die transfizierten BA/F3-huFLT3 zeigen und die eine negative Reaktion mit Zellen der nicht transfizierten BA/F3-Zellinie zeigen.

Die Erfindung betrifft auch die Verwendung des erfindungsgemäβen monoklonalen Antikörpers gegen das FLT3/FLK2-Rezeptorprotein zur Herstellung eines Mittels zur diagnostischen und/oder therapeutischen Behandlung von Tumoren, insbesondere von malignen hämatopoetischen Zellen wie z.B.: lymphoiden und myeloischen Leukämiezellen.

Es wurde nämlich überraschenderweise gefunden, daß die meisten malignen hämatopoetischen Zellen, wie z.B. lymphoide und myeloische Leukämiezellen, einen vergleichsweise hohen Gehalt an FLT3/FLK2-Rezeptorprotein aufweisen. Ein erfindungsgemäßer Antikörper, der mit einem Nachweismittel, beispielsweise einem radioaktiven Marker, gekoppelt ist, bindet dieses Nachweismittel indirekt an die betreffenden Zellen und ermöglicht damit den direkten Nachweis dieser Zellen, beispielsweise mit röntgendiagnostischen /szintigraphischen Methoden. Damit ist eine sehr frühe Diagnose unter Umständen sogar in vivo möglich.

In entsprechender Art und Weise kann der Antikörper mit einem therapeutisch wirksamen Mittel gekoppelt sein und dadurch eine direkte und gezielte Beeinflussung oder gar Eliminierung von FLT3/FLK2-Rezeptorprotein tragenden Zellen, insbesondere Leukämiezellen, ermöglichen.

Um die therapeutische und/oder diagnostische Applikation des erfindungsgemäßen Antikörpers zu erleichtern, kann der Antikörper mit entsprechend geeigneten Hilfssubstanzen in einer pharmazeutischen Zubereitung vermischt sein. Die Erfindung betrifft deshalb auch ein pharmazeutisches Mittel zur diagnostischen und/oder therapeutischen Behandlung von Tumoren, das einen Antikörper enthält, wie er von den unter der Nummer DSM ACC2249 bei der Deutschen Sammlung von Mikroorganismen und Zellkulturen GmbH (DSMZ) hinterlegten Hybridomzellen produziert und freigesetzt wird.

Mit einem erfindungsgemäßen Antikörper können humanes FLT3/FLK2-Rezeptorprotein tragende Zellen aus einer Suspension verschiedenartiger (humaner) Zellen mit den im Stand der Technik bekannten Testverfahren, wie z.B. dem Enzyme linked immunosorbent assay, kurz ELISA, oder dem Radioimmunoassay, kurz RIA, nachgewiesen werden. Die vorliegende Erfindung betrifft deshalb auch einen Kit zum Nachweis von humanem FLT3/FLK2-Rezeptorprotein, der einen monoklonalen Antikörper umfaßt, der von den unter der Nummer DSM ACC2249 bei der Deutschen Sammlung von Mikroorganismen und Zellkulturen GmbH (DSMZ) hinterlegten Hybridomzellen erzeugt wird.

Im Rahmen der vorliegenden Erfindung wurde überraschenderweise gefunden, daß der monoklonale Antikörper mit der Bezeichnung 4G8B4B12, der von den unter der Nummer DSM ACC2249 bei der Deutschen Sammlung von Mikroorganismen und Zellkulturen GmbH (DSMZ) hinterlegten Hybridomzellen erzeugt wird, an humane Stammzellen bindet.

Die Erfindung betrifft deshalb auch die Verwendung des Antikörpers 4G8B4B12, zum Nachweis von hämatopoetischen Zellen, sowie einen Kit zum Nachweis von hämatopoetischen Zellen, der den Antikörper 4G8B4B12, enthält. Dadurch ist es möglich, undifferenzierte CD34⁺ Subpopulationen aufzutrennen und für funktionelle Analysen Zellen aus dem Knochenmark zu selektieren und aufzureinigen.

Weitere Vorteile ergeben sich aus der nachstehenden Beschreibung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird im folgenden anhand von Anwendungs- und Ausführungsbeispielen unter Heranziehung der Figuren näher erläutert.

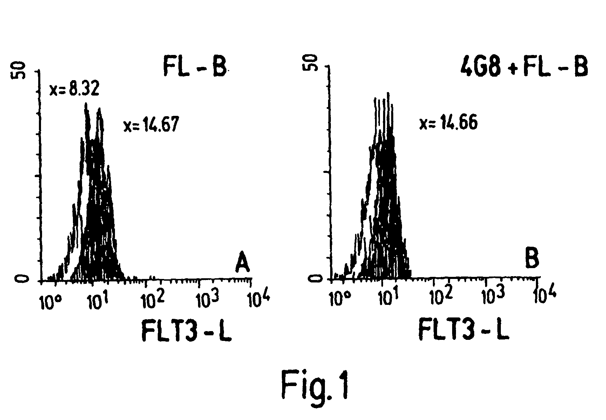

- Fig. 1: umfaßt das Histogramm A einer Durchflußzytometermessung von 4G1-Zellen, die mit Ligand ohne Antikörper inkubiert wurden, und das Histogramm B einer Durchflußzytometermessung von 4G1-Zellen, die mit Ligand und Antikörper 4G8B4B12 (dort mit 4G8 bezeichnet) inkubiert wurden; und

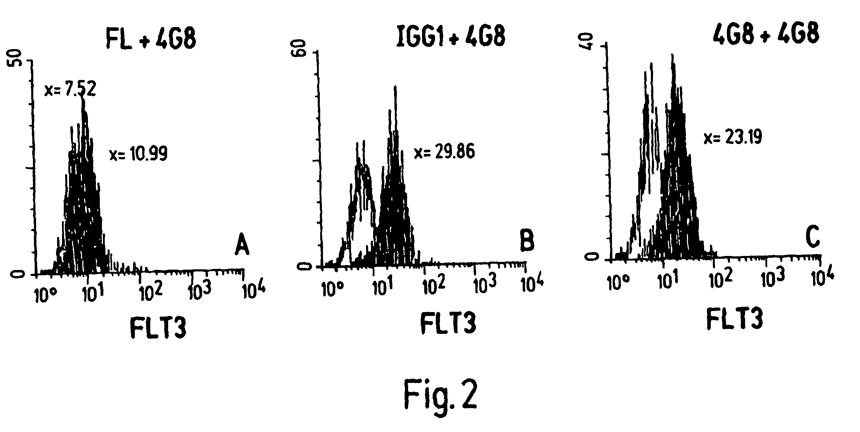

- Fig. 2: umfaßt drei Histogramme von Durchflußzytometermessungen an BV-173-Zellen, die bei B mit einem Kontrollantikörper vorinkubiert und mit dem Antikörper 4G8B4B12 (dort 4G8) und dem Ligand nach inkubiert wurden, bei C direkt mit dem erfindungsgemäßen Antikörper 4G8B4B12 (dort 4G8) und dem Ligand inkubiert wurden und bei A allein mit dem Ligand inkubiert wurden.

### Beispiel 1: Herstellung und Charakterisierung von monoklonalen Antikörpern gegen das FLT3/FLK2-Rezeptorprotein

Als Antigen werden Zellen einer BA/F3 Mauszellinie verwendet, die zuvor mit der kompletten cDNA für das humane FLT3/FLK2-Rezeptorprotein transfiziert wurde (BA/F3-huFLT3).

Um diese Zellen zu gewinnen, wurde die komplette Codesequenz der cDNA für das humane FLT3/FLK2-Rezeptorprotein in den Säugerexpressionsvektor pCD-SRα kloniert und zusammen mit einem Plasmid, das die G418-Resistenz überträgt, in die Interleukin-3-abhängigen BA/F3-Zellen eingebracht.

Transfizierte Zellen, die den FLT3/FLK2-Rezeptor exprimieren, wurden aufgrund ihrer G418-Resistenz und anhand ihres Wachstums in Gegenwart von rekombiniertem FLT3/FLK2-Rezeptorprotein-Ligand (FL) (Lyman et al., Cell 75:1157, 1993) isoliert und angereichert. Ein Klon, 4G1, wurde nach Grenzverdünnung ausgewählt und auf seine Fähigkeit zur Expression von FLT3/FLK2-Rezeptorprotein überprüft. Dazu wurden die Zellen dieses Klons 4G1 zunächst mit radioaktivem Methionin (³⁵S) markiert und anschließend mit einem Kaninchen-Antiserum, das gegen die Insertionssequenz der Tyrosin-Kinase-Domäne des FLT3/FLK2-Rezeptorproteins gerichtet ist, inkubiert. Das dadurch erhaltene Immunpräzipitat wurde mittels Polyacrylamid-Gelelektrophorese analysiert.

Die auf diese Weise als BA/F3-huFLT3 bestätigten Zellen des Klons 4G1 werden als Antigen eingesetzt.

Acht Wochen alte Balb/c-Mäuse werden zweimal in Intervallen von 10 Tagen intraperitoneal mit 10⁷ Zellen des Klons 4G1 immunisiert. Vier Tage vor der Fusion werden 5 x 10⁵ Zellen direkt in die Milz appliziert, um die Immunantwort zu verstärken.

Die Antikörper-Bildung im Mausorganismus wird dadurch überprüft, daß das Blutserum des betreffenden Tieres in dem dem Fachmann geläufigen ELISA-Test auf Bindungseigenschaften mit dem Antigen untersucht wird.

Nach ca. 3 Wochen werden die Lymphozyten des erfolgreich immunisierten Tieres gewonnen, indem die Milz herausoperiert und zu einer Zellsuspension zerkleinert wird.

Die suspendierten Milzzellen werden in Anwesenheit von Polyethylenglykol mit Myelomzellen des bekannten Stammes SP2/0 fusioniert. Die Fusionskultur wird in Hypoxanthin-, Aminopterinund Thymidin-(HAT-)haltigem Medium, hier in HAT-RPMI-1640, kultiviert, in dem sich nur Hybridzellen vermehren können, da diese sowohl die Eigenschaft der Myelomzellen zur unbegrenzten Teilungsfähigkeit als auch die Eigenschaft der Antikörper produzierenden Lymphozyten zum Wachstum in HAT-haltigem Medium haben.

Nach der Fusion werden die Zellen in Napfplatten ausplattiert und bei 37°C, 5 % Co₂ inkubiert.

Die Kulturüberstände werden nach 10-14 Tagen auf Zellen des Klons 4G1 im Durchflußzytometer gescreent. In einem zweiten Schritt werden die Überstände auf Reaktivität mit Zellen der Zellinie Ba/F3 getestet, da diese keine Antigene exprimieren. Hybridome, die Antikörper mit einer Spezifität für 4G1-Zellen produzieren, werden ausgewählt und nach dem bekannten Grenzverdünnungsverfahren vereinzelt und kultiviert, d.h. kloniert.

Positiv reagierende Hybridomzellkulturen werden weiter kultiviert, die Antikörper angereichert, gereinigt und charakterisiert.

Nach der obigen Screening-Strategie wurde der monoklonale Antikörper 4G8B4B12 erhalten. Der IgG1 Isotyp wurde über ein Phycoerythrin (PE)-konjugiertes Anti-Isotyp-spezifisches Antiserum durch indirekte Immunfluoreszenz mittels Durchflußzytometrie bestimmt.

Die Produktion, Reinigung und Charakterisierung der Antikörper erfolgte mit den in Fachkreisen allgemein bekannten Methoden.

Der Antikörper 4G8B4B12, der von den unter der Nummer DSM ACC2249 bei der Deutschen Sammlung von Mikroorganismen und Zellkulturen GmbH (DSMZ) hinterlegten Hybridomzellen erzeugt wird, weist die folgenden charakteristischen Merkmale auf:

| | |
|---|---|
| Immunglobulinklasse | IgG1 |
| spezifische Bindungsaffinität an | humanes FLT3/FLK2-Rezeptorprotein (extrazelluläre Domäne) |

### Beispiel 2: Identifizierung des von dem monoklonalen Antikörper 4G8B4B12 erkannten Antigens.

Die Identifizierung des Antigens erfolgte durch Bindungsversuche an Zellen mit und ohne FLT3/FLK2-Rezeptor.

Eine Probe (A) mit Mauszellen der gemäß Beispiel 1 erzeugten Zellinie BA/F3-huFLT3, die mit der humanen cDNA für den FLT3/FLK2-Rezeptor transfiziert sind, wurde mit dem erfindungsgemäßen Antikörper 4G8B4B12 inkubiert.

Zur Kontrolle wurde eine Probe (B) mit Mauszellen der nicht transfizierten Zellinie BA/F3 ebenfalls mit dem Antikörper 4G8B4B12 in derselben Konzentration wie Probe (A) inkubiert.

Beide Proben wurden mit einem anti-IgG1-PE-Antiserum markiert und anschließend im Durchflußzytometer analysiert.

Eine Bindung des Antikörpers 4G8B4B12 konnte ausschließlich bei Probe (A) nachgewiesen werden. Das heißt, daß der erfindungsgemäße Antikörper 4G8B4B12 spezifisch an diejenigen Zellen gebunden hat, die durch die cDNA für humanen FLT3/FLK2 -Rezeptor transformiert wurden und infolgedessen den humanen FLT3/FLK2-Rezeptor exprimieren.

### Beispiel 3: Identifizierung des monoklonalen Antikörpers 4G8B4B12 als nicht antagonistisch und leicht agonistisch wirkender Antikörper

Die durch die Bindung des Liganden initiierte Aktivierung des Rezeptors führt bei Tyrosin-Kinase-Rezeptoren, wie dem FLT3/FLK2-Rezeptor zunächst zur Bildung von Rezeptordimerkomplexen und deren Internalisierung in die Zelle. Manche Antikörper können den Liganden simulieren, d.h. sie wirken agonistisch, und führen deshalb ebenfalls zu einer solchen Rezeptordimer-Bildung und -Internalisierung (Bühring et al., Cancer Res. 53: 4424, 1993).

Eine Probe (A) 4G1-Zellen (gemäß Beispiel 1 gewonnen) wurde mit 200 ng/ml biotyniliertem FLT3 Ligand-Biotin und Streptavidin-Phycoerythrin (SA-PE) inkubiert.

Eine zweite Probe 4G1-Zellen (B) wurde zunächst 30 Minuten mit 7µg/ml Antikörper 4G8B4B12 inkubiert und anschließend wie Probe (A) behandelt.

Beide Proben wurden im Durchflußzytometer analysiert. Die dabei erhaltenen Histogramme sind in Fig. 1 dargestellt.

Beide Histogramme zeigen gleichstarke Signale. Das heißt, daß der erfindungsgemäße Antikörper 4G8B4B12 keinen hemmenden Einfluß auf die Bindung des Liganden hat und somit nicht antagonistisch wirkt (Bühring et al., Cancer Res 53:4424, 1993).

Zum Nachweis der agonistischen Reaktion wurde eine Probe (B) mit Zellen der hämatopoetischen Pro-B -Zellinie BV 173 (DSM ACC20; frei verfügbar) 2 Std. bei 37°C mit einem Kontroll-IgG1-Antikörper vorinkubiert, dann mit dem erfindungsgemäßen Antikörper 4G8B4B12 inkubiert und schließlich mit anti-IgG1-PE-Antiserum nachinkubiert.

Eine weitere Probe (C) wurde zuerst mit dem Antikörper 4G8B4B12 inkubiert und anschließend wie Probe (B) behandelt.

Eine Kontrollprobe (A) wurde zuerst mit 100 ng/ml FLT3/FLK2-Rezeptor-Ligand inkubiert und dann wie Probe (B) behandelt.

Alle drei Proben wurden im Durchflußzytometer analysiert. Die dabei erhaltenen Histogramme sind in Fig. 2 dargestellt. Das Histogramm C zeigt ein gegenüber dem Histogramm B um ca. 33% vermindertes Signal. Das heißt, daß der erfindungsgemäße Antikörper 4G8B4B12 die Internalisierung von einigen FLT3/FLK2-Rezeptoren bewirkt hat und damit leicht agonistisch (den Liganden simulierend) wirkt.

Das Histogramm A zeigt gegenüber dem Histogramm B, bedingt durch die naturgemäße Liganden induzierte Internalisierung der FLT3/FLK2-Rezeptoren, ein um 90% vermindertes Signal.

### Beispiel 4: Verwendung des monoklonalen Antikörpers 4G8B4B12 zum Nachweis von Subpopulationen der Blutstammzellen

Eine Probe frisch gewonnener Knochenmarkzellen wurde im Ficoll-Hypaque-Dichtegradienten aufgetrennt und die normalen, mononuklearen Knochenmarkzellen isoliert und angereichert.

Diese mononuklearen Knochenmarkzellen wurden dann mit einem PE-Konjugat des erfindungsgemäßen Antikörpers 4G8B4B12 sowie mit bekannten Fluorescein-Isothiocyanat (FITC)-konjugierten Antikörpern gegen die Antigene HLA-DR, CD34 bzw. CD62L von Stammzellen, CD33 von myeloiden Zellen, CD71 von erythroiden und Vorläuferzellen, CD10 bzw. CD19 von B-Zellen und CD3 bzw. CD7 von T-Zellen inkubiert.

Die Zellen wurden im Durchflußzytometer analysiert.

Die Analyseergebnisse zeigen:
- eine starke Koexpression von FLT3/FLK2 mit den Stammzellmarkern CD34, HLA-DR und CD62L (55% bzw. 100% bzw. 60%),
- eine schwächere Koexpression mit dem myeloiden Zellmarker CD33 und den erythroiden und Vorläuferzellmarkern CD10 und CD19 (60% bzw. 20% bzw. 20%), und
- keine Koexpression mit den T-Zellmarkern CD7 und CD3.

Das bedeutet, daß mit dem erfindungsgemäßen Antikörper 4G8B4B12 Blutstammzellen sowie myeloide Zellen und Vorläuferzellen der B-Reihe identifiziert und insbesondere von T-Zellen unterschieden werden können.

Zur Charakterisierung von Subpopulationen der CD34⁺-Stammzellpopulation wurde eine 3-Farben-Analyse durchgeführt:

Hierzu wurden die mononuklearen Zellen mit einem PE-Konjugat des erfindungsgemäßen Antikörpers 4G8B4B12, einem bekannten FITC-konjugierten Antikörper gegen CD34 und einem bekannten Biotin-konjugierten Antikörper gegen CD117 inkubiert und schließlich im Durchflußzytometer analysiert.

Die erhaltenen Ergebnisse zeigen, daß 23% der CD34⁺-Zellen auch das Rezeptorprotein FLT3/FLK2 exprimieren und 50% der Zellen dieser Subpopulation auch das Protein CD117 aufweisen.

Untersuchungen an CD34⁺⁺- und CD34⁺⁺⁺-Zellen zeigen, daß die Subpopulation der FLT3/FLK2⁺ / CD117⁺-Zellen mit fallender CD34-Expression (von CD34⁺⁺⁺ über CD34⁺⁺ bis zu CD34⁺), das heißt mit fortschreitender Entwicklung der Blutstammzellen, zahlenmäßig abnimmt, während die Subpopulation der FLT3/FLK2⁺ / CD117 ⁻-Zellen anwächst.

Mit dem erfindungsgemäßen Antikörper 4G8B4B12 ist es somit möglich, zwei neue Subpopulationen der CD34-positiven Population der Blutstammzellen zu isolieren, nämlich die Subpopulation FLT3/FLK2⁺ / CD117⁺ und die Subpopulation FLT3/FLK2⁺ / CD117 ⁻.

### Beispiel 5: Verwendung des monoklonalen Antikörpers 4G8B4B12 zum Nachweis von leukämischen Blasten der myeloischen und B-lymphozytären Reihe

Aus Knochenmark oder peripherem Blut von Leukämie-Patienten wurde eine reine (>90%) Population von leukämischen Blasten mittels Zentrifugation in einem Ficoll-Gradienten gewonnen.

Die Zellen wurden gemäß der Französisch-Amerikanisch-Britischen (FAB)-Klassifikation (Bennet et al., Ann. Intern. Med. 103: 620, 1985) klassifiziert und diese Klassifizierung durch phänotypische Analysen bestätigt.

Die Zellen wurden mit dem Antikörper 4G8B4B12 inkubiert, der gebundene Antikörper mit einem PE-konjugierten Anti-IgG1-Antiserum markiert und die Zellen im Durchflußzytometer analysiert.

In den meisten Blasten-Populationen von Patienten mit akuter myeloischer und/oder B-lymphozytärer Leukämie wurde eine positive Reaktion und damit das Vorhandensein von FLT3/FLK2-Rezeptorprotein nachgewiesen.

Ergänzend zu diesen Untersuchungen an frisch isolierten Leukämiezellen wurden Zellproben von megakaryoblastischen Zellinien, myeloid/monocytischen Zellinien und B-Zellinien getestet.

Die Zellen sämtlicher Proben wurden ebenfalls mit dem Antikörper 4G8B4B12 inkubiert, der gebundene Antikörper mit einem PE-konjugierten Anti-IgG1-Antiserum markiert und die Zellen im Durchflußzytometer analysiert.

Eine positive Reaktion und damit eine Expression von FLT3/FLK2-Rezeptorprotein wurde auf den meisten Zellinien der B-lymphozytären Reihe und auf wenigen der myeloischen Reihe gefunden.

Der erfindungsgemäße Antikörper 4G8B4B12 erweist sich somit als besonders geeignetes Nachweismittel für die Identifizierung von malignen hämatopoetischen Zellen der myeloischen und B-lymphozytären Reihe.

## Patentansprüche

1. Monoklonaler Antikörper, **dadurch gekennzeichnet, daß** er von Hybridomzellen produziert und freigesetzt wird, die unter der Nummer DSM ACC2249 bei der Deutschen Sammlung von Mikroorganismen und Zellkulturen GmbH (DSMZ) gemäß dem Budapester Vertrag hinterlegt worden sind und die Bezeichnung 4G8B4B12 tragen.

2. Hybridomzellen, **dadurch gekennzeichnet, daß** sie unter der Nummer DSM ACC2249 bei der Deutschen Sammlung von Mikroorganismen und Zellkulturen GmbH (DSMZ) gemäß dem Budapester Vertrag hinterlegt worden sind und die Bezeichnung 4G8B4B12 tragen.

3. Verwendung eines Antikörpers nach Anspruch 1 zur Herstellung eines Mittels zur diagnostischen Behandlung von Tumoren, insbesondere von malignen hämatopoetischen Zellen.

4. Verwendung eines Antikörpers nach Anspruch 1 zur Herstellung eines Mittels zur therapeutischen Behandlung von Tumoren, insbesondere von malignen hämatopoetischen Zellen.

5. Pharmazeutisches Mittel zur diagnostischen Behandlung von Tumoren, **dadurch gekennzeichnet, daß** es einen Antikörper gemäß Anspruch 1 enthält.

6. Pharmazeutisches Mittel nach Anspruch 5, **dadurch gekennzeichnet, daß** der Antikörper an ein zellulär zielgerichtetes Therapeutikum gekoppelt ist.

7. Pharmazeutisches Mittel zur therapeutischen Behandlung von Tumoren, **dadurch gekennzeichnet, daß** es einen Antikörper nach Anspruch 1 enthält.

8. Pharmazeutisches Mittel nach Anspruch 7, **dadurch gekennzeichnet, daß** der Antikörper an ein zellulär zielgerichtetes Diagnostikum gekoppelt ist.

9. Kit zum Nachweis von FLT3/FLK2-Rezeptorprotein, **dadurch gekennzeichnet, daß** er einen Antikörper nach Anspruch 1 enthält.

10. Verwendung eines Antikörpers nach Anspruch 1 zum Nachweis von hämatopoetischen Zellen in einer Probe.

11. Kit zum Nachweis von hämatopoetischen Zellen, **dadurch gekennzeichnet, daß** er einen Antikörper nach Anspruch 1 enthält.

12. Verwendung eines Antikörpers nach Anspruch 1 zum Nachweis von Stammzellsubpopulationen in einer Probe.

## Claims

1. A monoclonal antibody, **characterized in that** it is produced and released by hybridoma cells that are deposited under No. DSM ACC2249 at the German Collection of Microorganisms and Cell Cultures Ltd. (DSMZ) in accordance with the Budapest Treaty, and designated 4G8B4B12.

2. Hybridoma cells, **characterized in that** they are deposited under No. DSM ACC2249 at the German Collection of Microorganisms and Cell Cultures Ltd., DSMZ, in accordance with the Budapest Treaty and designated 4G8B4B12.

3. Use of an antibody according to claim 1 for the preparation of a medicament for diagnostic treatment of tumors, especially of malignant hematopoietic cells.

4. Use of an antibody according to claim 1 for the preparation of a medicament for therapeutic treatment of tumors, especially of malignant hematopoietic cells.

5. A pharmaceutical means for diagnostic treatment of tumors, **characterized in that** it comprises an antibody according to claim 1.

6. The pharmaceutical means according to claim 5, **characterized in that** the antibody is coupled to a cellularly targeted therapeutical agent.

7. A pharmaceutical means for therapeutic treatment of tumors, **characterized in that** it comprises an antibody according to claim 1.

8. The pharmaceutical means according to claim 7, **characterized in that** the antibody is coupled to a cellularly targeted diagnostic agent.

9. Kit for the detection of FLT3/FLK2 receptor protein, **characterized in that** it comprises an antibody according to claim 1.

10. Use of an antibody according to claim 1 for detection of hematopoietic cells in a sample.

11. Kit for the detection of hematopoietic cells, **characterized in that** it comprises an antibody according to claim 1.

12. Use of an antibody according to claim 1 for detection of stem cell subpopulations in a sample.

## Revendications

1. Anticorps monoclonal, **caractérisé en ce qu'**il est produit et libéré par des cellules hybridomes qui ont été déposées sous te numéro DSM ACC2249 auprès de la Deutsche Sammlung von Mikroorganismen und Zellkulturen GmbH (DSMZ) conformément au Traité de Budapest et qui portent la désignation 4G8B4B12.

2. Cellules hybridomes, **caractérisées en ce qu'**elles ont été déposées sous le numéro DSM ACC2249 auprès de la Deutsche Sammlung von Mikroorganismen und Zellkulturen GmbH (DSMZ) conformément au Traité de Budapest et qu'elles portent la désignation 4G8B4B12.

3. Utilisation d'un anticorps selon la revendication 1 en vue de fabriquer un médicament pour le traitement diagnostique des tumeurs, en particulier des cellules hématopoïétiques malignes.

4. Utilisation d'un anticorps selon la revendication 1 en vue de fabriquer un médicament pour le traitement thérapeutique des tumeurs, en particulier des cellules hématopoïétiques malignes.

5. Médicament pour le traitement diagnostique de tumeurs, **caractérisé en ce qu'**il comprend un anticorps selon la revendication 1.

6. Médicament selon la revendication 5, **caractérisé en ce que** l'anticorps est couplé à un agent thérapeutique ciblant des cellules.

7. Médicament pour le traitement thérapeutique de tumeurs, **caractérisé en ce qu'**il comporte un anticorps selon la revendication 1.

8. Médicament selon la revendication 7, **caractérisé en ce que** l'anticorps est couplé à un agent de diagnostic ciblant des cellules.

9. Kit pour déceler des protéines réceptrices FLT3/FLK2, **caractérisé en ce qu'**il comprend un anticorps selon la revendication 1.

10. Utilisation d'un anticorps selon la revendication 1 pour déceler des cellules hématopoïétiques dans un échantillon.

11. Kit pour déceler des cellules hématopoïétiques, **caractérisé en ce qu'**il comprend un anticorps selon la revendication 1.

12. Utilisation d'un anticorps selon la revendication 1 pour déceler des sous-populations de cellules souches dans un échantillon.
